Europäisches Patentamt

European Patent Office

Office européen des brevets

(11) Publication number : **0 588 505 A2**

# (12) EUROPEAN PATENT APPLICATION

(21) Application number : 93306552.6

(22) Date of filing : 19.08.93

(51) Int. Cl.⁵ : **G01N 33/00, G01N 30/00**

(30) Priority : **01.09.92 US 938812**

(43) Date of publication of application :
**23.03.94 Bulletin 94/12**

(84) Designated Contracting States :
**DE FR GB**

(71) Applicant : **CHROMATOFAST**
**912 North Main Street**
**Ann Arbor, Michigan 48104 (US)**

(72) Inventor : **Klemp, Mark Allen**
**5152 South Morrish Road No.48**
**Swartz Creek, Michigan 48473 (US)**

(74) Representative : **Senior, Alan Murray**
**J.A. KEMP & CO., 14 South Square Gray's Inn**
**London WC1R 5LX (GB)**

(54) Instrument for measuring non-methane organic gases in gas samples.

(57)  An analytical system for measuring the concentration of non-methane organic gases within a test sample including methane and non-methane component groups. The system incorporates a trap in the form of a porous layer open tubular (PLOT) column which the sample mixture is directed through and which traps the NMOG group through adsorption. The methane group passes through the trap and is vented to atmosphere or evaluated using a detector. After trapping of the NMOG group, the flow direction through the column is reversed and the column temperature is increased to desorb the NMOG group which is evaluated using a detector such as a flame ionization detector (FID).

FIG-1

EP 0 588 505 A2

## BACKGROUND AND SUMMARY OF THE INVENTION

This invention relates to an organic compound analytical instrument, and in particular, to one capable of providing a measurement of the concentration of non-methane organic gases (NMOG) in samples containing methane such as internal combustion engine exhaust gases.

Those concerned with environmental emissions from sources such as internal combustion engines are often interested in quantifying the total concentration of certain compounds within the exhaust gas. In some evaluations, rather than requiring speciation of the mixture, the total concentration of NMOG emissions as a lumped parameter is desired. The distinction between methane and NMOG relates to differences in environmental impact attributed to these different groups of gases.

Various techniques are currently used for determining NMOG concentrations. One approach obtains the value of NMOG as a difference in the measurements of total hydrocarbons and methane using a flame ionization detector (FID). Several problems are associated with the current test methods. As methane can be a significant proportion of exhaust composition, small errors in the measurement of either methane or total hydrocarbon levels result in large errors in the estimation of NMOG levels. In fact, it is not uncommon for this evaluation technique to result in yields of negative NMOG emissions. In addition, the current method requires large volumes of dilution air to be mixed with the exhaust gas, and samples are stored in so-called "Tedlar" sample bags before analysis. The purpose of dilution air is to prevent significant water condensation on the walls of the Tedlar bags. The use of Tedlar bags is problematic with certain compounds found in exhaust gas, and it has been found that the dilution air can have levels of NMOG exceeding that found in the raw gas under certain circumstances. The use of Tedlar bags further raises concerns with the test reliability, reproduceability and stability, and provides a media for absorption and interactions of certain compounds. Particularly in the case of oxygenated organic compounds, reactivity with the walls of the Tedlar bags is a known problem. Moreover, current processes are very time consuming to carry out, which is a particular disadvantage in engine development applications.

The accuracy limitations of current procedures for NMOG determination are especially of concern in the view of increasingly stringent vehicle emission requirements. Legislation by the Federal Government (Federal Clean Air Act) and the California Air Research Board (CARB) have defined further limitations on vehicle emissions to improve air quality. These new mandates require that vehicles sold must be designed to meet stringent emissions standards which are a fraction of the presently accepted levels. In addition to regulatory compliance evaluation, instruments for NMOG determination are also useful by automotive manufacturers during their development activities.

Other evaluation procedures have been proposed and considered for methane and NMOG measurement using a trap in which NMOG components are collected and later desorbed for evaluation using an FID. In one device, a packed column for absorbing NMOG components is used to separate out the methane, and thereafter, the trapped NMOG is desorbed. Traps based on absorption, however, have the disadvantage of having a long desorption time which lengthens measurement time and limits accuracy due to the difficulty in integrating the output.

In view of the foregoing, there is a need to provide an analytical instrument which provides a fast and accurate measurement of NMOG concentrations. The instrument in accordance with this invention provides such evaluation within several seconds rather than tens of minutes as in the case of prior art devices. The instrument of this invention further provides a means of direct sampling which eliminates the need for sample collection bags and large volumes of dilution air. The instrument of this invention operates on the basis of a trap which enables separation of methane from NMOG. The separator comprises a porous layer open tubular (PLOT) column which, when held at or below ambient temperature, permits a group of gases including methane to pass through whereas a second group of NMOG components are adsorbed within the column. The unseparated methane gas group can be evaluated using a detector such as an FID or can be vented. After the methane gas group has been transported through the column, fluid flow direction through the column is reversed and the column temperature is increased, causing the retained NMOG to be desorbed and directed through a detector such as a FID. By integrating the output of the detector over time, a quantitative evaluation of the concentration of NMOG components is provided.

Additional benefits and advantages of the present invention will become apparent to those skilled in the art to which this invention relates from the subsequent description of the preferred embodiments and the appended claims, taken in conjunction with the accompanying drawings.

## BRIEF DESCRIPTION OF THE DRAWINGS

Figure 1 is a schematic illustration of the analysis instrument in accordance with this invention.

Figure 2 is a representative chromatogram from a detector providing a measurement of methane concentration using the instrument of Figure 1.

Figure 3 is a representative chromatogram from a detector providing a measurement of NMOG concentration using the instrument of Figure 1.

## DETAILED DESCRIPTION OF THE INVENTION

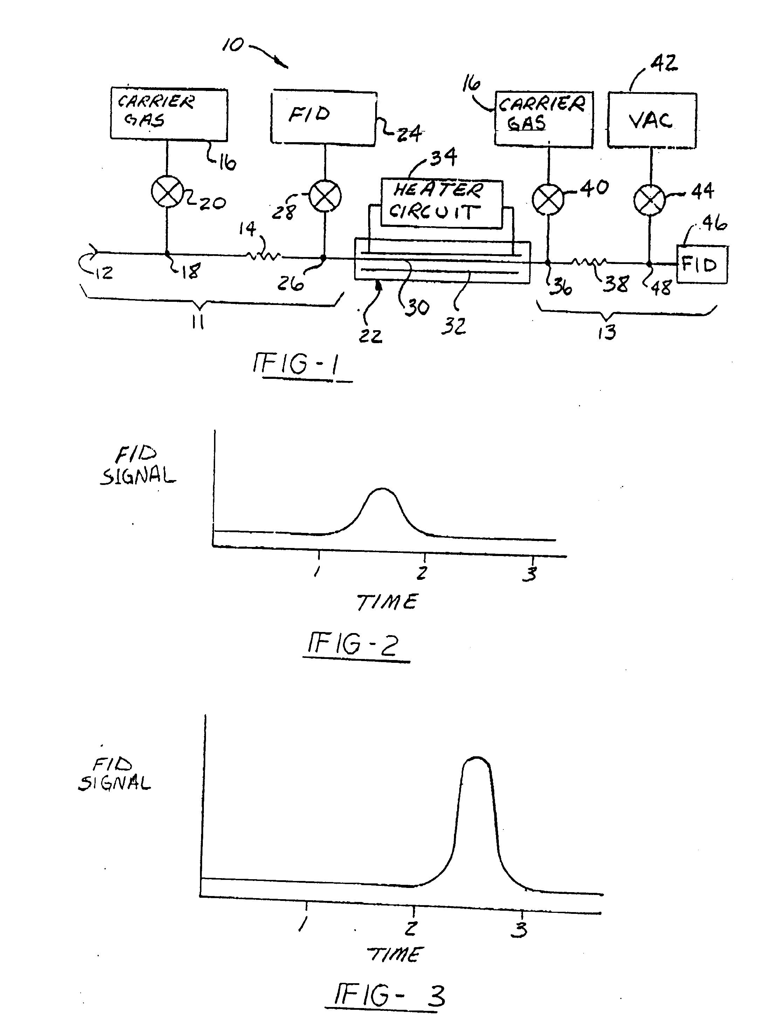

An analysis system in accordance with this invention is shown in schematic fashion in Figure 1 and is designated there by reference number 10. In the Figure, solid lines connecting system elements indicate fluid flow conduits. System 10 includes a first branch 11 of fluid flow elements on the left-hand side of the Figure, with a second branch 13 at the right-hand side of the Figure. First branch 11 includes sample inlet 12 which is the point at which a sample to be evaluated is introduced, such as internal combustion engine exhaust gases. Inlet 12 is connected to a fluid restrictor 14 which is provided for adjusting fluid flow rates through the system. A carrier gas source 16 supplying a gas such as hydrogen is connected between sample inlet 12 and restrictor 14 at tee connector 18 through on/off valve 20. First branch 11 is also connected to trap 22. A detector 24 which can take various forms but preferably is a flame ionization detector (FID) 24 is connected between restrictor 14 and trap 22 at tee connection 26.

Trap 22 is comprised of an elongated column of the porous layer open tubular (PLOT) type. PLOT column 30 has an inside coating of a stationary phase material such as aluminum oxide (AlO) which adsorbs a group of gases referred to as NMOG, while permitting a second group of gases including methane and so-called permanent gases (nitrogen, oxygen, carbon dioxide) to pass through the column. Other PLOT column configurations and stationary phases could also be used, including the following commercially available products: PoraPLOT Q™, PoraPLOT U™, and CarboPLOT ™ columns. Column 30 is surround by heater tube 32 which is connected to an electric heater circuit 34 which permits the temperature of column 30 to be rapidly increased for the desorption of trapped NMOG, as will be described in more detail. The opposite end of column 30 is connected to second branch 13 which includes fluid restrictor 38 which like restrictor 14, serves to control fluid flow rates. Carrier gas source 16 is connected at the connection 36 between column 30 and restrictor 38 through on/off valve 40. Vacuum source 42 is connected through on/off valve 44 between restrictor 38 and detector 46 at tee connector 48. Detector 46 like detector 24, is preferably an FID. Detector 46 is needed only when a quantitative evaluation of the methane group is desired. Where such a measurement is not needed, tee connector 48 can be vented to atmosphere.

An example of analysis system 10 would employ the following specific components and parameters for the analysis of internal combustion engine exhaust gases:

| ELEMENT NUMBER | ELEMENT NAME | SPECIFICATION/PARAMETER |
| --- | --- | --- |
| 14 and 38 | Restrictor | 0.1mm fused silica glass capillary tubing, 30cm long |
| 16 | Carrier Gas Source | Hydrogen |
| 20,28,40,44 | Valves | Low dead-volume On/off solenoid controlled valves of the type commonly used in gas chromatography instruments |
| 30 | Column | 15cm long fused silica glass capillary, 0.32mm ID with AlO stationary phase coating |
| 32 | Heater Tube | Stainless Stell Tubing |
| 34 | Heater Circuit | Capacitive-Discharge circuit as described in U.S. Patent No. 5,096,471, herein incorporated by reference |

Operation of analysis system 10 will now be described with particular reference to the Figures. At the beginning of an analysis procedure, exhaust gas or another gas sample for evaluation is delivered to sample inlet 12 while valves 20, 28, and 40 are closed, and valve 44 is open. Vacuum source 42 draws the sample into first branch 11 and toward column 30. As soon as the sample has past beyond tee connection 18, the vacuum source 42 is disconnected by closing valve 44. Thereafter, the carrier gas source 16 drives the sample into column 30 by opening valve 20.

The temperature of column 30 is set to allow the methane group of gases to pass through with very little retention while adsorbing the NMOG group onto the stationary phase of column 30. Accordingly, column 30 acts as a separation column for the methane and NMOG groups. In order to provide separation, the temperature of column 30 needs to be held at a "trapping" temperature within a predetermined range, which is believe to extend from ambient temperatures (eg. 20°C) to sub-ambient temperatures down to -100°C and perhaps lower, depending on the particular configuration of system 10 and especially column 30. Where an ambient trapping temperature is used, the trapping temperature can be provided by exposing column 30 to ambient air. Where cooler trapping temperatures are needed, a bath of cooled water or a cold gas (eg. nitrogen) can be used to cool column 30.

Once the methane gas group has passed through column 30 and past tee connection 36, carrier gas source 16 is applied to tee connection 36 by opening valve 40, while valve 20 remains open. This action produces a splitting of the flow of carrier gas at tee connection 36 in which a fraction of the carrier gas continues to drive the methane gas group through restrictor 38 and through FID 46 where its presence is detected and a quantitative measurement is provided. Figure 2 provides a representative output from FID 46 which can be integrated for a measure of the concentration of the methane gas group. The balance of carrier gas 16 flows into column 30 which causes the direction of fluid flow through the column to be reversed; namely, toward FID 24. Simultaneous with the flow reversal, heater circuit 34 is activated, causing the temperature of column 30 to be increased rapidly to a desorption temperature above the trapping temperature. The desorption temperature would be above ambient (20°C) and may be 200°C or higher, depending on the characteristics of system 10. This temperature increase causes the NMOG group components retained by column 30 to be desorbed. By opening valve 28, the NMOG components which are desorbed pass to FID 24 where they are detected and integrated. Figure 3 provides a representative output of the desolved NMOG group from FID 24. The flow reversal through column 30 also provide backflushing for clearing the column for a subsequent sample evaluation. After integration at FID 24, valves 20, 28 and 40 are again closed, enabling the next sample to be introduced into the system.

While the above description constitutes the preferred embodiments of the present invention, it will be appreciated that the invention is susceptible of modification, variation and change without departing from the proper scope and fair meaning of the accompanying claims.

## Claims

1. An analysis system for the measurement of a concentration of a gaseous sample including a non-methane organic gases group and a methane gases group including methane, comprising:

   a porous layer open tubular column which, when at a trapping temperature, allows the methane gases group to pass through said porous layer open tubular column while adsorbing the non-methane organic gases group, and at an elevated desorption temperature above said trapping temperature, causes the nonmethane organic gases group to be desorbed,

   temperature control means for causing said porous layer open tubular column to be at said trapping temperature, or at said elevated desorption temperature,

   detector means operatively connected to said porous layer open tubular column for measuring the concentration of the non-methane organic gases group after the non-methane organic gases group is desorbed, and

   fluid circuit means for causing the gaseous sample to flow through said porous layer open tubular column in a first direction when said porous layer open tubular column is at said trapping temperature causing the non-methane organic gases group to be adsorbed, and thereafter causing the desorbed non-methane organic gases group to flow in a second opposite direction through said porous layer open tubular column when said column is at said elevated desorption temperature.

2. An analysis system according to claim 1, wherein said detector means provides a measurement of the concentration of the methane gases group and comprises a flame ionisation detector receiving the methane gases group after passing through said column in said first direction.

3. An analysis system for measurement of a concentration of a gaseous sample including a non-methane organic gases group and a methane gases group including methane, comprising:

   a porous layer open tubular column defining opposite first and second ends which, when at trapping temperature, selectively allows the methane gases group to pass through said porous layer open tubular column while adsorbing the non-methane organic gases group, and at a desorption temperature above said trapping temperature, causes the non-methane organic gases group to be desorbed,

temperature control means for selectively causing said porous layer open tubular column to be held at said trapping temperature or at said desorption temperature,

detector means operatively connected to said porous layer open tubular column for measuring the concentration of the non-methane organic gases group after the non-methane organic gases group is desorbed,

a fluid circuit having an inlet for receiving the gaseous sample and having a first branch connected to said porous layer open tubular column first end and connected to said detector means, said fluid circuit further having a second branch connected to said porous layer open tubular column second end, and

valve means within said fluid circuit for causing the gaseous sample to pass into said porous layer open tubular column from said first circuit branch toward said second circuit branch in a first flow direction when said porous layer open tubular column is at said trapping temperature causing the non-methane organic gases group to be adsorbed, and thereafter causing the desorbed non-methane organic gases group to flow through said porous layer open tubular column toward said first circuit branch in a second opposite direction when said porous layer open tubular column is at said desorption temperature, causing the non-methane organic gases group to pass through said detector means where said concentration is measured.

4. An analysis system according to claim 3, wherein said detector means provides a measurement of the concentration of the methane gases group and comprises a flame ionisation detector connected to said second branch and receiving the methane gases group.

5. An analysis system according to any preceding claim, wherein said detector means comprises a flame ionisation detector receiving the non-methane organic gases group passing through said porous layer open tubular column in said second direction.

6. An analysis system according to any preceding claim, wherein said analysis system is adapted to determine the concentration of a non-methane organic gases group of an exhaust gas sample of an internal combustion engine.

7. An analysis system according to any preceding claim, wherein said temperature control means comprises an electrical resistance heater.

8. An analysis system according to any preceding claim, wherein said porous layer open tubular column includes an aluminium oxide stationary phase material.

9. An analysis system according to any preceding claim, wherein said temperature control means is adapted to maintain a trapping temperature less than 20°C.

10. An analysis system according to claim 9, wherein said temperature control means is adapted to maintain a trapping temperature greater than -100°C.

11. An analysis system according to any preceding claim, wherein said temperature control means is adapted to maintain an elevated desorption temperature greater than 20°C.

12. An analysis system according to claim 11, wherein said temperature control means is adapted to maintain an elevated desorption temperature less than 200°C.

FIG-1

FIG-2

FIG-3